# EUROPÄISCHE PATENTSCHRIFT

(11) **EP 3 034 100 B1**
(45) Veröffentlichungstag und Bekanntmachung des Hinweises auf die Patenterteilung: **24.01.2018**
(21) Anmeldenummer: 15200712.6
(22) Anmeldetag: 17.12.2015
(51) Int. Cl.: A61L 2/20, F24F 3/16

(54) **DEKONTAMINATIONSANORDNUNG MIT VERSTELLBAREM KATALYSATOR SOWIE BETRIEBSVERFAHREN**
DECONTAMINATION MODULE WITH MOVABLE CATALYST AND PROCESS USING SAID MODULE
MODULE DE DÉCONTAMINATION AVEC UN CATALYSEUR MOBILE ET PROCEDE UTILISANT LEDIT MODULE

(30) Priorität: 18.12.2014 DE 102014119029
(43) Veröffentlichungstag der Anmeldung: 22.06.2016
(60) Teilanmeldung: 16198350.7
(73) Patentinhaber: Metall + Plastic GmbH, 78315 Radolfzell (DE)
(72) Erfinder: VON STENGLIN, Christoph, 78315 Radolfzell (DE)
(74) Vertreter: Patentanwälte Behrmann Wagner PartG mbB

(56) Entgegenhaltungen:
- WO-A1-2010/078081
- WO-A1-2014/079779

## Beschreibung

Die Erfindung betrifft eine Dekontaminationsanordnung (Vorrichtung), insbesondere für pharmatechnische Anwendungen, gemäß dem Oberbegriff des Anspruchs 1 mit einem zu dekontaminierendem Raum, insbesondere einem Isolatorraum, mit einer, bevorzugt einen Dekontaminationsmitteldampferzeuger umfassenden, Dekontaminationseinrichtung, mit einem Gebläse zum Erzeugen eines Gasluftvolumenstroms und mit einem Katalysator zum chemischen Aufspalten des Dekontaminationsmittels, wobei in einem ersten Betriebszustand der Dekontaminationsanordnung ein größerer Anteil des Gasvolumenstroms als in einen zweiten Betriebszustand, insbesondere der gesamte Gasvolumenstrom, durch den Katalysator hindurch förderbar ist.

Gattungsbildende Dekontaminationsanordnungen zeichnen sich dadurch aus, dass eine, bevorzugt einen Verdampfer für Wasserstoffperoxyd umfassende Dekontaminationseinrichtung Dekontaminationsmittel zum Dekontaminieren eines zu dekontaminierenden Raums (in der Regel ein Isolatorraum) aufweist. Nach einer ausreichenden Einwirkzeit des Dekontaminationsmittels muss der mit dem Dekontaminationsmittel in Kontakt gekommene Bereich, insbesondere der vorgenannte zu dekontaminierende Raum freigespült werden. Bei früheren Dekontaminationsanordnungen wurde dieses Freispülen dadurch realisiert, dass Frischluft durch den zu dekontaminierenden Raum gefördert wurde. Bei der aus der WO 2011/085735 A1 und der EP 2 534 426 B1 bekannten Vorrichtung wird die Freispülzeit in vorteilhafter Weise dadurch verkürzt, dass beim Freispülen das Dekontaminationsmittel mittels des Gebläses durch einen Katalysator gefördert wird. Ein Abbau von Dekontaminationsmittel während einer Konditionierungsphase, in der Dekontaminationsmittel zugeführt wird und während einer Einwirkphase, während deren das Dekontaminationsmittel seine dekontaminierende Wirkung entfaltet wird dadurch verhindert, dass das als Umluftgebläse ausgebildete Gebläse zumindest über den größten Zeitabschnitt der Konditionierungsphase bzw. der Einwirkphase ausgeschaltet wird oder bleibt.

Aus der US 6,010,400 A ist ebenfalls eine Dekontaminationsanordnung bekannt. Hier wird das Dekontaminationsmittel durch einen mit Katalysator getränkten Filter hindurchgefördert.

Aus der JP 2006-116095 A ist es bekannt, Dekontaminationsmittel zu dessen Aufspaltung durch einen Katalysator zu fördern. Um einen Abbau des Dekontaminationsmittel während der Konditionierungs- und Einwirkphase zu vermeiden, wird bei der bekannten Vorrichtung der Gasvolumenstrom während vorgenannter Phasen über einen Bypass am Katalysator vorbei gefördert. Ein ähnliches Prinzip ist in der JP 2011-167405 A beschrieben.

Von der Anmelderin wurde in der WO 2014/079779 A1 eine Lösung beschrieben, bei der ein Katalysator und ein Gebläse ortsfest relativ zueinander angeordnet sind und ein Faltenbalg in einen ersten Betriebszustand von dem Gebläse angesaugt oder weggedrückt wird, um eine gasleitende Verbindung zwischen dem Gebläse und dem Katalysator herzustellen, so dass in dem ersten Betriebszustand der Gasvolumenstrom zum Abbau des Dekontaminationsmittels durch den Katalysator hindurchgefördert wird. In einem zweiten Betriebszustand, währenddessen das Gebläse mit einer niedrigeren Drehzahl betrieben wird, so dass die mittels des Gebläses erzielte Saug- oder Druckkraft nicht ausreicht, um einen Bypass zu schließen, wird der Katalysator von dem Gasvolumenstrom umströmt.

Insbesondere die zuletzt beschriebene Ausführungsform mit mittels des Gebläses sowohl relativ zum Katalysator als auch zu dem Gebläse verstellbaren Faltenbalg hat sich bewehrt. Es bestehen jedoch Bestrebungen hin zu einem noch robusteren und einfacheren Ausbau einer Dekontaminationsanordnung, die insbesondere dazu ausgelegt ist, den Katalysator im Umlufterzeugerraum aufzuweisen, wobei mittels des Gebläses der Gasvolumenstrom als Umluftvolumenstrom im Kreis zwischen dem Umlufterzeugerraum und dem zu isolierenden Raum gefördert wird.

Aus der WO 2010/078081 A1 ist eine mobile Dekontaminationsvorrichtung bekannt, die dazu dient, um nach einem Waschvorgang chirurgische Instrumente in einen entsprechenden Raum zu desinfizieren. Zu diesem Zweck ist die Dekontaminationsvorrichtung in den Raum einschiebbar. Die bekannte Vorrichtung umfasst mehrere, jeweils in einem Rohr angeordnete Gebläse, wobei in Bereichen hinter den Gebläsen in den Rohrleitungen Katalysatoren angeordnet sind, die aus den Rohrleitungen heraus verstellt werden können.

Ausgehend von dem vorgenannten Stand der Technik liegt der Erfindung daher die Aufgabe zugrunde, eine alternative Dekontaminationseinrichtung anzugeben, bei welcher mit einfachen Mitteln gewährleistet ist, dass im ersten Betriebszustand ein größerer Gasvolumenstrom durch den Katalysator förderbar ist als in einem zweiten Gasbetriebszustand, um in letzterem zumindest weitgehend eine Aufspaltung (Abbau) von Dekontaminationsmittel zu verhindern. Die Vorrichtung soll dabei einfach und robust sein und möglichst wenig bewegte Teile aufweisen. Bevorzugt soll die Dekontaminationsvorrichtung die Anordnung des Katalysators in einem oberhalb des zu dekontaminierenden Raums ausgebildeten Umlufterzeugerraum ermöglichen. Ferner besteht die Aufgabe darin, ein Betriebsverfahren für eine entsprechend verbesserte Dekontaminationsanordnung anzugeben.

Diese Aufgabe wird hinsichtlich der Dekontaminationsanordnung mit den Merkmalen des Anspruchs 1 gelöst.

Hinsichtlich des Verfahrens wird die Aufgabe mit den Merkmalen des Anspruchs 8 gelöst.

Vorteilhafte Weiterbildungen der Erfindung sind in den Unteransprüchen angegeben.

Zur Vermeidung von Wiederholungen sollen vorrichtungsgemäß offenbarte Merkmale als verfahrensgemäß offenbart gelten und beanspruchbar sein. Ebenso sollen verfahrensgemäß offenbarte Merkmale auch als vorrichtungsgemäß offenbart gelten und beanspruchbar sein.

Der Erfindung liegt der Gedanke zugrunde, trotz seiner Größe und seines Gewichtes den Katalysator selbst, insbesondere zusammen mit einem Katalysatorgehäuse relativ zu dem Gehäuse verstellbar anzuordnen, und zwar zwischen einer ersten und einer zweiten Relativposition, wobei der Katalysator in der ersten Relativposition so angeordnet ist, dass ein größter Anteil des Gasvolumenstroms, insbesondere der gesamte Gasvolumenstrom zum Zwecke des Abbaus bzw. des Aufspaltens des Dekontaminationsmittels durch den Katalysator strömt, während die zweite Relativposition so gewählt ist, dass in dieser zumindest deutlich weniger Gas, vorzugsweise kein Gas mehr durch den Katalysator strömt, sondern dieser bevorzugt zumindest weitgehend nur noch von dem Gasvolumenstrom umströmt, d.h. gebypasst wird. In Abgrenzung zu der aus der WO 2014/079779 A1 der Anmelderin bekannten Dekontaminationsanordnung ist bei der erfindungsgemäßen Dekontaminationsanordnung der Katalysator, insbesondere zusammen mit einem Katalysatorgehäuse Bestandteil von Gasleitmitteln, die den größten Teil des Gasvolumenstroms im ersten Betriebszustand durch den Katalysator und im zweiten Betriebszustand am Katalysator vorbeileiten. Die erfindungsgemäße Dekontaminationsanordnung rückt ab von dem bisher geltenden Grundprinzip der statischen Anordnung sowohl des mindestens einen Gebläses als auch des mindestens einen Katalysators, indem als ein Erfindungsgedanke der Katalysator ortsvariabel, d.h. verstellbar angeordnet ist zwischen einer ersten und einer zweiten Relativposition zu dem Gehäuse, wobei der Katalysator in der ersten Relativposition so gasleitend angebunden bzw. mit dem Gebläse verbunden ist, dass ein größerer Teil eines Gasvolumenstroms, insbesondere der gesamte Gasvolumenstrom durch den Katalysator strömt als im zweiten Betriebszustand. Durch die erfindungsgemäße Ausgestaltung der Dekontaminationsanordnung kann auf von dem Katalysator und einem Katalysatorgehäuse separate bzw. relativ zu einem Katalysator verstellbare Faltenbalganordnungen sowie auf Ventile, wie im Stand der Technik eingesetzte Klappenventile, verzichtet werden, woraus ein besonders einfacher und robuster Aufbau der Dekontaminationsanordnung resultiert. Darüber hinaus resultiert aus der verstellbaren Anordnung des Katalysators bzw. aus dem daraus resultierenden Verzicht auf im Stand der Technik notwenige bewegte Teile, wie Faltenbalge und/oder Ventile eine Reduzierung eines abriebbedingten Partikeleintrags, hier von sogenannten non-viable (nicht lebensfähigen) Partikeln, die wiederum Träger für Mikroorganismen sein können, was erhebliche Vorteile für den hier insbesondere Einsatz in der Pharmaindustrie mit sich bringt.

Bevorzugt kommt ein Katalysator zum Einsatz, bei dem das Katalysatormaterial, insbesondere MnO₂, als Schüttgut in einem Behältnis (Katalysatorgehäuse) vorliegt. Noch weiter bevorzugt ist der Katalysator auf eine Trägerstruktur, bevorzugt eine starre Trägerstruktur aufgebracht, insbesondere auf einen offenporigen Metallschaum, insbesondere ein Aluminium-Nickel- oder Chromnickelschaum, wie dieser in der WO 2011/085735 A1 der Anmelderin beschrieben ist. Im letztgenannten Fall kann der Katalysator in einer Umhausung angeordnet sein, die zusammen mit dem Träger verstellbar ist. In diesem Fall bildet die Umhausung ein Isolatorgehäuse - alternativ kann auf eine solche Umhausung verzichtet werden. Dann stellt das Trägermaterial selbst das Katalysatorgehäuse dar.

Besonders zweckmäßig ist es, wenn im Gasvolumenstrom, insbesondere in Strömungsrichtung hinter dem Katalysator ein Hochleistungsschwebstofffilter, insbesondere ein Hepa-Filter und/oder Ulpa-Filter angeordnet sind/ist.

Ganz besonders zweckmäßig ist es, wenn der Katalysator in einem oberhalb des zu dekontaminierenden Raums angeordneten Umlufterzeugerraum angeordnet ist, aus dem mittels des Gebläses Gas angesaugt und in Richtung zu dem dekontaminierenden Raum gefördert wird. Der Gasvolumenstrom wird bevorzugt als Umluftvolumenstrom zwischen dem Umlufterzeugerraum und dem zu dekontaminierenden Raum im Kreislauf mittels des mindestens einen Gebläses gefördert. Besonders zweckmäßig ist es, wenn sich zwischen dem Umlufterzeugerraum und dem zu dekontaminierenden Raum ein Zwischenraum befindet, der von dem zu dekontaminierenden Raum über eine Membran zur Vergleichmäßigung des Luftstroms getrennt ist.

Im Hinblick auf die Position zur Einleitung des mittels der Dekontaminationseinrichtung bereitgestellten Dekontaminationsmittels, insbesondere ein Dekontaminationsmitteldampf, weiter bevorzugt ein Wasserstoffperoxyddampf, gibt es unterschiedliche Möglichkeiten. So ist es denkbar, das Dekontaminationsmittel unmittelbar in den dekontaminierenden Raum einzubringen. Bevorzugt ist eine Ausführungsform, bei der das Dekontaminationsmittel, insbesondere der Dekontaminationsmitteldampf in den Umlufterzeugerraum eingebracht wird und zu Zwecken der gleichmäßigen Verteilung im zweiten Betriebszustand mittels des Gebläses, relativ zu dem der Katalysator verstellbar angeordnet ist, im Kreislauf gefördert wird.

Im Hinblick auf die Anzahl von vorzusehenden Gebläsen und Katalysatoren gibt es unterschiedliche Möglichkeiten. Grundsätzlich ist es möglich, mehrere Gebläse zum Erzeugen des Gasvolumenstroms vorzusehen, wobei dann der gesamte Gasvolumenstrom gemäß einer ersten Alternative dann durch einen einzigen Katalysator in seiner ersten Relativposition förderbar ist. Denkbar ist es auch, dass mehrere Gebläse kombiniert sind mit mehreren Katalysatoren, d.h. dass der von den Gebläsen erzeugte Gasvolumenstrom auf mehrere Katalysatoren in deren jeweils erster Relativposition aufteilbar ist. Ganz besonders bevorzugt ist eine Ausführungsform, bei der jedem der, vorzugsweise jeweils als Umluftgebläse ausgebildeten und angeordneten Gebläse ein Katalysator zugeordnet ist, durch welchen der von den jeweiligen Gebläse erzeugte Gasvolumenstrom in der jeweiligen ersten Relativposition gefördert wird. Dies hat den entscheidenden Vorteil, dass aufgrund der Aufteilung der gesamten Katalysatorflächen auf mehrere Katalysatoren insgesamt eine größere Reaktions- bzw. Katalysatorfläche zur Verfügung gestellt werden kann und somit die Freispülzeit auf ein Minimum reduziert wird. Auch ist es möglich, nur ein einziger Gebläse, insbesondere Umluftgebläse vorzusehen und den von diesem erzeugten Gasvolumenstrom durch nur einen einzigen Katalysator in seiner ersten Relativposition zur fördern oder alternativ durch mehrere Katalysatoren, die dann in ihrer ersten Relativposition entsprechend gasleitend mit dem einzigen Gebläse verbunden sind.

Bei einer erfindungsgemäßen Ausführungsform der Dekontaminationsanordnung ist vorgesehen, dass der Katalysator mit Hilfe von Federmitteln relativ zu einer Halteeinrichtung federnd gelagert ist, derart, dass der Katalysator entgegen einer Rückstellkraft der Federmittel in die erste Relativposition verstellbar ist. Mit anderen Worten kann dann eine Rückstellung in die zweite Relativposition, in welcher bevorzugt ein Grossteil des Gasvolumenstroms am Katalysator vorbeiströmt, dieser also gebypasst wird automatisch bzw. federkraftunterstützt bzw. federkraftaktuiert erfolgen.

Erfindungsgemäß ist weiter vorgesehen, dass zum, bevorzugt rein translatorischen, Verstellen des Katalysators in die erste Relativposition entgegen der Federkraft der vorgenannten Federmittel die Saugkraft des mindestens einen Gebläses genutzt wird. Mit anderen Worten sind die Federmittel derart ausgelegt bzw. dimensioniert, dass der Katalysator im ersten Betriebszustand, insbesondere ausschließlich, durch eine Saugkraft des Gebläses, d.h. von dem Gebläse in die erste Relativposition ansaugbar ist bzw. angesaugt wird.

Damit die Gewichtskraft des Katalysators in die gleiche Richtung wirkt wie die Verstellbewegung von der zweiten in die erste Relativposition und somit die Gewichtskraft die Bewegung unterstützt und das Gebläse somit im Wesentlichen nur die Rückstellkraft der Federmittel überwinden muss, ist in Weiterbildung der Erfindung mit Vorteil vorgesehen, dass das Gebläse den Katalysator entgegen dessen Gewichtskraftrichtung ansaugend angeordnet ist. Bevorzugt ist der Katalysator hierzu oberhalb des Gebläses angeordnet, jedenfalls oberhalb einer diesem zugeordneten Ansaugöffnung des Gebläses. Als besonders zweckmäßig hat es sich dabei herausgestellt, wenn die Halteeinrichtung, gegenüber welcher der Katalysator federnd gelagert ist gleichzeitig das Gebläse trägt - in diesem Fall werden die Federmittel beim Ansaugen des Katalysators in die erste Relativposition von dem Katalysator auf Druck beaufschlagt - d.h. die Federn sind bevorzugt so angeordnet, dass sie den Katalysator zurück in die zweite Relativposition drücken, wobei selbstverständlich auch eine alternative Ausführungsform realisierbar ist, wonach die Federn hierzu auf Zug belastbar angeordnet sind.

Um mit demselben Gebläse im ersten Betriebszustand Gas durch den Katalysator hindurch und im zweiten Betriebszustand an diesem vorbeifördern zu können und gleichzeitig das Gebläse zur Aktuierung bzw. zum Verstellen des Katalysators von der zweiten in die erste Relativposition nutzen zu können, ist in Weiterbildung der Erfindung mit Vorteil vorgesehen, dass das Gebläse von Steuermitteln in den vorgenannten Betriebszuständen mit einer unterschiedlichen Drehzahl betrieben wird. Um den Katalysator aus der zweiten in die erste Relativposition ansaugen zu können wird das Gebläse von den Steuermitteln im ersten Betriebszustand mit einer höheren Drehzahl betrieben als im zweiten Betriebszustand, wobei die Drehzahlen so gewählt sind, dass im ersten Betriebszustand eine ausreichend große Saugkraft resultiert, um den Katalysator, insbesondere entgegen seiner Gewichtskraftrichtung ansaugen und insbesondere die Rückstellkraft der Federmittel überwinden zu können, wohingegen die Drehzahl im zweiten Betriebszustand so gewählt ist, dass zumindest ein Teil, vorzugsweise der Großteil des Gasvolumenstroms am Katalysator vorbeiströmen kann und dieser zumindest nicht bis in die erste Relativposition verstellt wird.

Wie bereits angedeutet ist dem Katalysator bevorzugt eine zusammen mit diesem von der zweiten in die erste Relativposition verstellbare Dichtfläche zugeordnet, die bevorzugt von einem Gehäuseabschnitt des Katalysators gebildet ist. Bei der Dichtfläche muss es nicht um eine elastomere Dichtfläche handeln - in diesem Fall wirkt die Dichtfläche in der ersten Relativposition bevorzugt mit einer elastomeren Gegenfläche im Bereich einer Ansaugöffnung des Gebläses zusammen. Es ist jedoch auch denkbar, auf jegliches Elastomermaterial zu verzichten und somit ggf. Leckagegasvolumenströme bei in der ersten Relativposition befindlichem Katalysator am Katalysator vorbei zu tolerieren.

In der zweiten Relativposition sind das Gebläse und der Katalysator so weit voneinander beabstandet, dass zwischen dem Katalysator, insbesondere dem fest mit dem Katalysator gekoppelten bzw. gemeinsam mit diesem verstellbaren Gehäuse und einer Ansaugöffnung des Gebläses ein ausreichender Abstand (Spalt) besteht, durch welchen der Gasvolumenstrom im zweiten Betriebszustand strömt und somit den Katalysator bypasst.

Besonders zweckmäßig ist eine Ausführungsform, bei der der Katalysator im Umlufterzeugerraum angeordnet ist. Besonders zweckmäßig dabei ist es, wenn mehrere Gebläse vorgesehen sind, denen jeweils ein Katalysator zugeordnet ist, wobei die Katalysatoren jeweils zwischen einer zweiten Relativposition und einer ersten Relativposition verstellbar sind, entweder durch von mindestens einen von den Gebläsen separaten Aktuator oder mittels des mindestens einen Gebläses durch Ansaugen. Es ist jedoch auch eine Ausführungsform realisierbar, wenn auch weniger bevorzugt, bei der ein einziger Katalysator relativ zu den Gebläsen verstellbar ist, dann insbesondere mittels eines separaten Verstellantriebes, und zwar bevorzugt im ersten Betriebszustand gegen eine Druckleitungsöffnung, die bevorzugt in den Umlufterzeugerraum mündet und durch die von dem einen Gebläse oder den mehreren Gebläse Gas aus dem zu dekontaminierenden Raum in den Umlufterzeugerraum rückströmt. Diese Ausführungsform hat jedoch den Nachteil, dass entweder ein sehr großer und damit vergleichsweise schwer zu bewegender Katalysator vorgesehen werden muss oder, dass die Katalysatorfläche entsprechend gering ist.

Die Erfindung führt auch auf ein Verfahren zum Betreiben einer wie zuvor beschriebenen ausgebildeten Dekontaminationsanordnung, wobei erfindungsgemäß der Katalysator, insbesondere zusammen mit einem Katalysatorgehäuse, relativ zu dem Gebläse zwischen einer ersten und einer zweiten Relativposition verstellt wird, und dass in der ersten Relativposition ein größerer Anteil des Gasvolumenstroms durch den Katalysator hindurchströmt als in der zweiten Relativposition. Bevorzugt strömt in der ersten Relativposition der gesamte Gasvolumenstrom durch den Katalysator.

Erfindungsgemäß ist vorgesehen, dass die Verstellbewegung des Katalysators durch mindestens ein dem Katalysator zugeordnetes Gebläse aktuiert wird, indem nämlich die Saugkraft des Gebläses im ersten Betriebszustand benutzt wird, um den Katalysator in die erste Relativposition, insbesondere in zumindest mittelbarer Anlage an eine Ansaugöffnung des Gebläses zu bewegen. Hierzu ist die Drehzahl des Gebläses im ersten Betriebszustand bevorzugt entsprechend höher als im zweiten Betriebszustand.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1: einen möglichen Aufbau einer nach dem Konzept der Erfindung ausgebildeten Dekontaminationsanordnung in einer stark schematisierten Darstellung,
- Fig. 2: eine perspektivische Ansicht eines Details der Dekontaminationsanordnung gemäß Fig. 1, aus der die Anordnung eines Gebläses mit einen diesen zugeordneten Katalysator zu ersehen ist,
- Fig. 3: das Detail aus Fig. 2 in einem ersten Betriebszustand der die Dekontaminationsanordnung, bei welcher der Großteil des von dem Gebläse erzeugte Gasvolumenstrom durch den Katalysator gesaugt wird, und
- Fig. 4: einen zweiten Betriebszustand, in dem der Großteil des Dekontaminationsmittels am Katalysator vorbei gesaugt wird.

In den Figuren sind gleiche Elemente und Elemente mit der gleichen Funktion mit den gleichen Bezugszeichen gekennzeichnet.

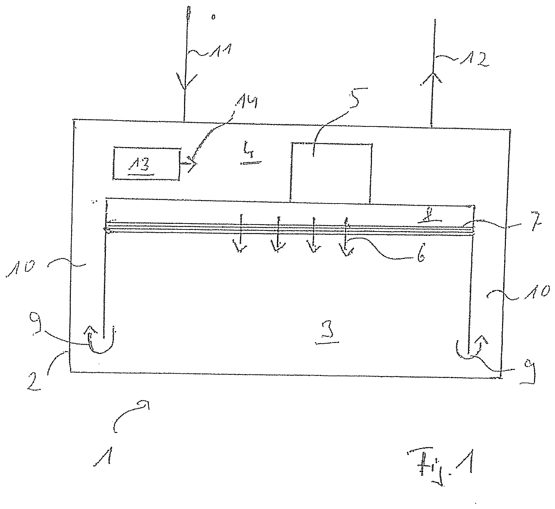

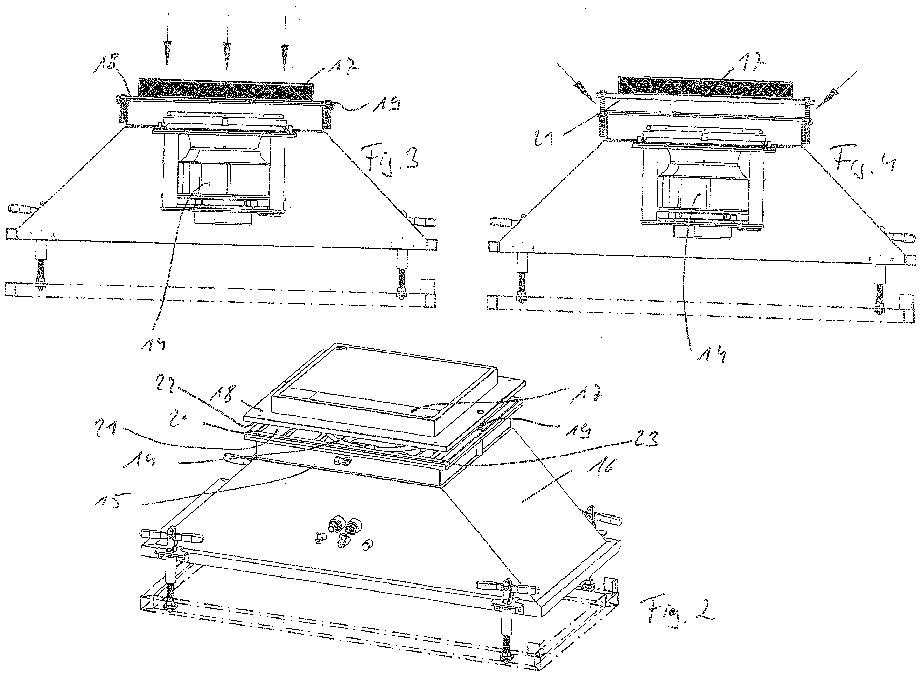

In Fig. 1 ist eine Dekontaminationsanordnung 1, umfassend einen Isolator 2 gezeigt. In diesem ist ein zu dekontaminierender Raum 3, beispielsweise ein Manipulatorraum vorgesehen. Oberhalb des zu dekontaminierenden Raums befindet sich ein Umlufterzeugerraum 4 mit einem als Umluftgebläse dienenden Gebläse, welches Teil einer "Blackbox" 5 ist, deren mögliche Ausgestaltungen anhand der Fig. 2a bis 3b im Folgenden noch erläutert werden. Teil der Blackbox 5 sind auch ein Katalysator zum Abbau von Dekontaminationsmittel sowie Gasleitmittel, die zwischen zwei Betriebszuständen umschaltbar sind. Fakultativ, jedoch bevorzugt sind auch Teil der Blackbox 5, wie später noch erläutert werden wird, Filtermittel, insbesondere in Form mindestens eines Hochleistungsschwebstofffilters, beispielsweise eines HEPA- und/oder ULPA Filters.

Um eine insgesamt größere Menge an Gasvolumen pro Zeiteinheit fördern und auch wieder von dem Dekontaminationsmittel befreien zu können ist es realisierbar und bevorzugt, mehrere Blackbox-Einheiten nebeneinander oberhalb eines später noch zu erläuternden Zwischenraums 8 anzuordnen, also mehrere Gebläse, denen jeweils ein relativ zu diesem ein verstellbarer Katalysator zugeordnet ist.

Das mindestens eine Gebläse ist so ausgelegt, dass mit diesem eine gleichmäßige Luftströmung 6 von oben nach unten in den zu dekontaminierenden Raum während des bestimmungsgemäßen Betriebs mit einer Strömungsgeschwindigkeit von etwa 0,45m/s erzeugbar ist. Das Gebläse erzeugt dabei zunächst eine turbulente Strömung, die mit einer Membran 7, ggf. in Kombination mit einem Hochleistungsschwebstofffilter in eine laminare Strömung umgewandelt wird. Der von dem Gebläse erzeugte Luftstrom gelangt in einen Zwischenraum 8, der nach unten von der vorerwähnten Membran 7 begrenzt ist. Die Membran 7 ist beispielsweise von einem Siebdruckgewebe gebildet. Die aus der Membran 7 nach unten strömende laminare Luftströmung gelangt in den zu dekontaminierenden Raum 3, strömt dort nach unten und dann in Pfeilrichtung 9 durch zwischen Doppelglasscheiben gebildete Strömungskanäle 10 innerhalb des Isolators 2 nach oben zurück in den Umlufterzeugerraum, aus dem sie von dem Gebläse wieder angesaugt wird.

In dem Isolator 2, in dem konkreten Ausführungsbeispiel in dem oberen Umlufterzeugerraum 4 mündet eine Frischluftleitung 11 für konditionierte Luft, insbesondere während des Freispülvorgangs. Aus dem Isolator 2, in dem konkreten Ausführungsbeispiel aus dem Umlufterzeugerraum 4 mündet eine Abluftleitung 12 hinaus. Der Frischluftleitung 11 ist eine nicht dargestellte Temperier- und Entfeuchtungseinrichtung vor- bzw. zugeordnet.

Innerhalb des Umlufterzeugerraums 4 befindet sich eine Dekontaminationseinrichtung 13 mit Verdampfer zum Verdampfen von Dekontaminationsmittel, insbesondere von Wasserstoffperoxid. Die Dekontaminationseinrichtung 13 weist einen Auslass 14 auf, der in den Umlufterzeugerraum 4 ausmündet und welcher innerhalb des Umlufterzeugerraums 4 beabstandet ist von einer in Fig. 1 nicht gezeigten Ansaugöffnung für das Gebläse.

Selbstverständlich kann die Dekontaminationseinrichtung 13 auch außerhalb des Umlufterzeugerraums 4 angeordnet sein, wobei bevorzugt eine Auslassöffnung der Dekontaminationseinrichtung 13 in den Umlufterzeugerraum 4 mündet. Denkbar ist auch ein davon unterschiedlicher Ausmündungsort - beispielsweise in den zu dekontaminierenden Raum 3 hinein. Jedenfalls ist bevorzugt jedes der zur Anwendung kommenden und mit einem Katalysator gepaarten Gebläse ein Umluftgebläse, mit welchem ein Gasvolumenstrom in Kreislauf befördert wird, vorzugsweise zwischen einem Umlufterzeugerraum 4 und einem zu dekontaminierenden Raum 3, ganz besonders bevorzugt über die vorerwähnten Strömungskanäle 10.

Im Folgenden wird anhand der Fig. 2 bis 3 eine mögliche, bevorzugte Ausgestaltung der Anordnung im Umluftgebläse mit Katalysator erläutert.

In Fig. 2 ist ein als Umluftgebläse ausgebildetes und angeordnetes Gebläse 14 zu erkennen, mit welchem Gas aus dem Umlufterzeugerraum 4 angesaugt und nach unten in den Zwischenraum 8 gedrückt wird, durch welchen es (vgl. Fig. 1) in den zu dekontaminierenden Raum 3 und dann über die Strömungskanäle 10 zurück in den Umlufterzeugerraum 4 strömt.

Dem Gebläse 14 ist eine Halteeinrichtung 15 zugeordnet, mit welcher das Gebläse 14 ortsfest fixiert ist. An seinem Außenumfang ist die Halteeinrichtung 15 als eine Art sich in Strömungsrichtung des Gases öffnender Trichter 16 ausgebildet, in welchem das Gebläse 14 aufgehängt bzw. angeordnet ist.

Oberhalb des Gebläses 14 mit Abstand zu diesem befindet sich ein Katalysator zum chemischen Aufspalten von Wasserstoffperoxid. Dieser ist in einem Gehäuse 18 angeordnet, welches zusammen mit dem Katalysator 17 relativ zu dem Gebläse verstellbar angeordnet ist und zwar entgegen der Rückstellkraft von hier als Druckfedern ausgebildeten Federmitteln 19. Die Federmittel 19 beaufschlagen den Katalysator 17 entgegen seiner Gewichtskraftrichtung von dem Gebläse 14 weg - hier beispielhaft drückend.

Zu erkennen ist, dass zwischen dem Katalysator 17 bzw. dessen Gehäuse 18 und einer von dem Trichter 16 begrenzten Ansaugöffnung 20 des Gebläses 14 ein Abstand bzw. Spalt 21 gebildet ist, durch welchen Gas in einem später noch zu erläuternden zweiten Betriebszustand ansaugbar ist.

Die Federmittel 19 sind so dimensioniert, dass deren Federkraft nur etwas geringer ist als die Gewichtskraft des Katalysators 17 mit Gehäuse 18, so dass zum translatorischen Verstellen des Katalysators 17 zusammen mit seinem Gehäuse 18 nur ein geringer Kraftaufwand notwendig ist.

In Fig. 3 ist nun ein erster Betriebszustand der in Fig. 2 gezeigten Anordnung dargestellt. Zu erkennen ist, dass der Katalysator 17 nach unten in Richtung Gebläse 14 verstellt ist und zwar entgegen der Federkraft der Federmittel 19. In dem gezeigten Ausführungsbeispiel wurde bewusst auf einen extra Aktuator hierzu verzichtet - die Aktuator- bzw. Verstellfunktion wird von dem Gebläse 14 übernommen, welches in dem gezeigten Betriebszustand mit einer zu hohen Drehzahl betrieben wird, dass die resultierende Ansaugkraft ausreicht, den Katalysator 17 zusammen mit seinem Gehäuse 18 in Richtung Gebläse 14 zu bewegen und somit den Spalt 21 (vgl. Fig. 2) zumindest weitgehend zu schließen. In dem ersten Betriebszustand liegt bevorzugt das Gehäuse 18, zu dem auch eine Trägerplatte gehört, mit einer unterseitig daran angeordneten Dichtfläche 22 an eine ortsfest angeordneten Gegenfläche 23, hier der Halteeinrichtung 15 bzw. des Trichters 16 an.

Bei der gezeigten Konstruktion stützen sich die Federmittel 19 an der Halteeinrichtung 15 ab.

Wenn der Katalysator 17 mit seiner Dichtfläche 22 wie gezeigt in der ersten Relativposition zum Gebläse 14 angeordnet ist, muss der größte Teil des angesaugten Gasvolumenstroms, angedeutet über die drei Pfeile durch den Katalysator 17 strömen, so dass die Dekontaminationsmittel von dem Katalysator abgebaut werden kann.

In dem zweiten Betriebszustand, welcher in Fig. 4 gezeigt ist, ist die Gebläsedrehzahl niedriger, so dass der Spalt 21 gegeben ist. Der Katalysator 17 befindet sich in seiner zweiten Relativposition und Gas kann den Katalysator 17 umströmen und durch den Spalt 21 zum Gebläse 14 gelangen, so dass nur ein geringer Gasvolumenstrom überhaupt den Katalysator 17 durchströmt und damit einen Abbau von Dekontaminationsmittel weitgehend vermieden wird.

### Bezugszeichenliste

- 1: Dekontaminationsanordnung
- 2: Isolator
- 3: zu dekontaminierender Raum
- 4: Umlufterzeugerraum
- 5: "Blackbox"
- 6: laminare Luftströmung
- 7: Membran
- 8: Zwischenraum
- 9: Pfeilrichtungen (Strömungsrichtungen)
- 10: Strömungskanal
- 11: Frischluftleitung
- 12: Abluftleitung
- 13: Dekontaminationseinrichtung (Verdampfer)
- 14: Gebläse
- 15: Halteeinrichtung
- 16: Trichter
- 17: Katalysator
- 18: Gehäuse
- 19: Federmittel
- 20: Ansaugöffnung
- 21: Spalt
- 22: Dichtfläche
- 23: Gegenfläche

## Patentansprüche

1. Dekontaminationsanordnung (1) mit einem zu dekontaminierenden Raum (3) mit einer Dekontaminationseinrichtung (13) und mit einem Gebläse (14) zum Erzeugen eines Gasluftvolumenstroms und mit einem Katalysator (17) zum chemischen Aufspalten des Dekontaminationsmittels, wobei in einem ersten Betriebszustand ein größerer Anteil des Gasvolumenstroms als in einem zweiten Betriebszustand durch den Katalysator (17) hindurch förderbar ist, wobei der Katalysator (17) relativ zu dem Gebläse (14) zwischen einer ersten und einer zweiten Relativposition verstellbar ist und wobei in der ersten Relativposition ein größerer Anteil des Gasvolumenstroms, insbesondere der gesamte Gasvolumenstrom, durch den Katalysator (17) hindurch förderbar ist als in der zweiten Relativposition,
**dadurch gekennzeichnet,**
**dass** der Katalysator (17) mit Hilfe von Federmitteln (19) relativ zu einer Halteeinrichtung (15) derart federnd gelagert ist, dass der Katalysator (17) entgegen einer Rückstellkraft der Federmittel (19) in die erste Relativposition verstellbar ist und dass die Federmittel (19) derart ausgelegt sind, dass der Katalysator (17) im ersten Betriebszustand durch eine Saugkraft des Gebläses (14) von dem Gebläse (14) in die erste Relativposition ansaugbar ist.

2. Dekontaminationsanordnung nach Anspruch 1,
**dadurch gekennzeichnet,**
**dass** der Katalysator (17) in der ersten Relativposition gasleitend mit einer Ansaugöffnung des Gebläses (14) zusammenwirkend angeordnet ist oder alternativ mit einer Druckleitungsöffnung, durch die Gas mittels des Gebläses drückbar ist.

3. Dekontaminationsanordnung nach einem der vorhergehenden Ansprüche,
**dadurch gekennzeichnet,**
**dass** das Gebläse (14) den, bevorzugt oberhalb des Gebläses angeordneten, Katalysator (17) entgegen einer Schwerkraftrichtung des Katalysators (17) ansaugend angeordnet ist.

4. Dekontaminationsanordnung nach einem der vorhergehenden Ansprüche,
**dadurch gekennzeichnet,**
**dass** das Gebläse (14) mit Hilfe von Steuermitteln derart ansteuerbar ist, dass das Gebläse (14) im ersten Betriebszustand mit einer höheren Drehzahl betreibbar ist als im zweiten Betriebszustand, und dass die Drehzahl im ersten Betriebszustand so gewählt ist, dass durch die resultierende Saugkraft des Gebläses (14) der Katalysator (17) in die erste Relativposition verstellbar ist.

5. Dekontaminationsanordnung nach einem der vorhergehenden Ansprüche,
**dadurch gekennzeichnet,**
**dass** die eine zusammen mit dem Katalysator (17) verstellbare, bevorzugt an dem Katalysatorgehäuse (18) angeordnete,
Dichtfläche (22) in der ersten Relativposition dichtend an einer Ansaugöffnung (20) des Gebläses (14) anliegt.

6. Dekontaminationsanordnung nach einem der vorhergehenden Ansprüche,
**dadurch gekennzeichnet,**
**dass** der Katalysator (17) in der zweiten Relativposition derart von dem Gebläse (14) beabstandet ist, dass Gas durch einen von dem Katalysator (17) und dem Gebläse (14) gebildeten Spalt (21) von dem Gebläse (14) ansaugbar ist.

7. Dekontaminationsanordnung nach einem der vorhergehenden Ansprüche,
**dadurch gekennzeichnet,**
**dass** der Katalysator (17) in einem, insbesondere oberhalb des zu dekontaminierenden Raumes (3) angeordneten Umlufterzeugerraum (4) angeordnet ist.

8. Verfahren zum Betreiben einer Dekontaminationsanordnung nach einem der vorhergehenden Ansprüche,
**dadurch gekennzeichnet,**
**dass** der Katalysator (17) relativ zu dem Gebläse (14) zwischen einer ersten und einer zweiten Relativposition verstellt wird, und dass in der ersten Relativposition ein größerer Anteil des Gasvolumenstroms durch den Katalysator (17) hindurch strömt als in der zweiten Relativposition und dass der Katalysator (17) durch die Saugkraft des Gebläses (14) von der zweiten Relativposition in die erste Relativposition verstellt wird, entgegen der Rückstellkraft von Federmitteln (19).

## Claims

1. Decontamination assembly (1) with a room to be decontaminated with a decontamination means (13), and with a fan (14) for generating a gas air volume flow, and with a catalyst (17) for chemically breaking up the decontaminant, wherein a greater amount of the gas volume flow is conveyable through the catalyst (17) in a first operating state than in a second operating state, wherein the catalyst (17) is adjustable between a first and a second relative position, and wherein the catalyst (17) is adjustable to the fan (14) in a first and a second relative position, and wherein a greater amount of the gas volume flow, in particular the total gas volume flow is conveyable through the catalyst (17) in the first relative position than in the second relative position,
**characterized in that**
the catalyst (17), by means of spring means (19) is spring mounted relative to a holding device in such a way that the catalyst (17) is adjustable against a resetting force of the spring means (19) in the first relative position, and that the spring means (19) are configured in such a way that the catalyst (17) in the first operating state can be sucked in the first relative position by the fan through a suction force of the fan (14).

2. Decontamination assembly according to Claim 1,
**characterized in that**
the catalyst (17) is arranged to cooperate with a suction opening in a gas-conveying manner in the first relative position, or alternatively with a pressure line opening, through which gas can be pushed by means of the fan.

3. Decontamination assembly according to one of the preceding claims,
**characterized in that**
the fan (14) is arranged to suction the catalyst (17), preferably arranged above the fan, against a gravitational force of the catalyst (17).

4. Decontamination assembly according to one of the preceding claims,
**characterized in that**
the fan (14) is controllable by control means in such a way that the fan (14) is controllable in the first operating state with a higher rotational speed than in the second operating state, and that the rotational speed in the first operating state is selected in such a way that the catalyst (17) is adjustable in the first relative position through the resulting suction force of the fan (14).

5. Decontamination assembly according to one of the preceding claims,
**characterized in that**
the one sealing surface (22), adjustable together with the catalyst, preferably arranged on the catalyst housing (18), rests against the suction opening (20) of the fan (14) in a sealing manner in the first relative position.

6. Decontamination assembly according to one of the preceding claims,
**characterized in that**
the catalyst (17) is spaced from the fan (14) in the second relative position in such a way that the gas can be suctioned by the fan (14) through a gap (21) formed by the catalyst (17) and the fan (14).

7. Decontamination assembly according to one of the preceding claims,
**characterized in that**
the catalyst (17) is arranged in a circulating air generating room (4), in particular arranged above the room (3) to be decontaminated.

8. Method for operating a decontaminating assembly according to one of the preceding claims
**characterized in that**
the catalyst (17) will be adjusted relative to the fan (14) between a first and second relative position, and that, in the first relative position, a greater amount of the gas volume flow flows through the catalyst (17) than in the second relative position, and that the catalyst (17) is adjusted from the second relative position into the first relative position through the suction force of the fan (14) against a resetting force of the spring means (19).

## Revendications

1. Dispositif de décontamination (1) comprenant un espace (3) à décontaminer avec un dispositif de décontamination (13) et avec une soufflante (14) pour générer un débit volumique d'air et avec un catalyseur (17) pour la décomposition chimique de l'agent de décontamination, dans un premier état de fonctionnement, une plus grande proportion du débit volumique de gaz que dans un deuxième état de fonctionnement pouvant être refoulée à travers le catalyseur (17), le catalyseur (17) pouvant être déplacé par rapport à la soufflante (14) entre une première et une deuxième position relative et, dans la première position relative, une plus grande proportion du débit volumique de gaz que dans la deuxième position relative, en particulier la totalité du débit volumique de gaz, pouvant être refoulée à travers le catalyseur (17), **caractérisé en ce que** le catalyseur (17) est supporté élastiquement à l'aide de moyens de ressort (19) par rapport à un dispositif de retenue (15) de telle sorte que le catalyseur (17) puisse être déplacé dans la première position relative à l'encontre d'une force de rappel des moyens de ressort (19) et **en ce que** les moyens de ressort (19) sont conçus de telle sorte que le catalyseur (17), dans le premier état de fonctionnement, puisse être aspiré par la soufflante (14) dans la première position relative par une force d'aspiration de la soufflante (14).

2. Dispositif de décontamination selon la revendication 1, **caractérisé en ce que** le catalyseur (17), dans la première position relative, est disposé de manière à coopérer avec une ouverture d'aspiration de la soufflante (14) de manière à conduire du gaz ou, en variante, avec une ouverture de conduite de pression à travers laquelle du gaz peut être comprimé au moyen de la soufflante.

3. Dispositif de décontamination selon l'une quelconque des revendications précédentes, **caractérisé en ce que** la soufflante (14) est disposée de manière à aspirer le catalyseur (17) disposé de préférence au-dessus de la soufflante, à l'encontre d'un sens de pesanteur du catalyseur (17).

4. Dispositif de décontamination selon l'une quelconque des revendications précédentes, **caractérisé en ce que** la soufflante (14) peut être commandée à l'aide de moyens de commande de telle sorte que la soufflante (14), dans le premier état de fonctionnement, puisse être entraînée à une vitesse de rotation plus élevée que dans le deuxième état de fonctionnement et **en ce que** la vitesse de rotation dans le premier état de fonctionnement est choisie de telle sorte que par la force d'aspiration résultante de la soufflante (14), le catalyseur (17) puisse être déplacé dans la première position relative.

5. Dispositif de décontamination selon l'une quelconque des revendications précédentes, **caractérisé en ce que** l'une des surfaces d'étanchéité (22) pouvant être déplacée conjointement avec le catalyseur (17), de préférence disposée sur le boîtier de catalyseur (18), s'applique dans la première position relative hermétiquement contre une ouverture d'aspiration (20) de la soufflante (14).

6. Dispositif de décontamination selon l'une quelconque des revendications précédentes, **caractérisé en ce que** le catalyseur (17), dans la deuxième position relative, est espacé de la soufflante (14) de telle sorte que du gaz puisse être aspiré par la soufflante (14) à travers une fente (21) formée par le catalyseur (17) et la soufflante (14).

7. Dispositif de décontamination selon l'une quelconque des revendications précédentes, **caractérisé en ce que** le catalyseur (17) est disposé dans un espace de génération de circulation d'air (4) disposé notamment au-dessus de l'espace (3) à décontaminer.

8. Procédé pour faire fonctionner un dispositif de décontamination selon l'une quelconque des revendications précédentes, **caractérisé en ce que** le catalyseur (17) est déplacé par rapport à la soufflante (14) entre une première et une deuxième position relative et **en ce que**, dans la première position relative, une plus grande proportion du débit volumique de gaz que dans la deuxième position relative s'écoule à travers le catalyseur (17) et **en ce que** le catalyseur (17) est déplacé par la force d'aspiration de la soufflante (14) de la deuxième position relative dans la première position relative à l'encontre de la force de rappel de moyens de ressort (19).
